# EUROPEAN PATENT SPECIFICATION

(11) **EP 4 183 422 B1**
(45) Date of publication and mention of the grant of the patent: **02.12.2026**
(21) Application number: 23152297.0
(22) Date of filing: 27.03.2020
(51) Int. Cl.: A61L 2/08, A61J 1/10, B01L 9/00

(54) **CONTAINER FOR STERILISING FLEXIBLE BAGS**
BEHÄLTER ZUM STERILISIEREN VON FLEXIBLEN BEUTELN
RÉCIPIENT POUR STÉRILISER DES SACS SOUPLES

(30) Priority: 15.04.2019 EP 19382291
(43) Date of publication of application: 24.05.2023
(62) Document number(s) of the earlier application(s) in accordance with Art. 76 EPC: 20382242.4 / 3 725 336
(73) Proprietor: Grifols Worldwide Operations Limited, Clondalkin, Dublin 22 (IE)
(72) Inventor: BOIRA BONHORA, Jordi, 08150 Parets del Valles (Barcelona) (ES); ROURA SALIETTI, Carlos, 08150 Parets del Valles (Barcelona) (ES)
(74) Representative: Durán-Corretjer, S.L.P.

(56) References cited:
- US-A1- 2010 307 956
- US-A1- 2018 134 423

## Description

The present invention relates to the pharmaceutical industry, in particular to a new container for sterilising flexible bags for pharmaceutical use. More specifically, the present invention discloses a "nest" type container for sterilising flexible bags containing products derived from human plasma for therapeutic use.

Recently in the pharmaceutical industry in general and, in particular, in the industry of products obtained from human plasma, proof exists that plastic containers, in particular flexible plastic bags, are also useful as final packaging for hemoderivatives for several reasons: they are easy to shape, giving them great versatility and adaptability in their design, are rupture-resistant, ergonomic, and, due to their low density and weight, provide important costs savings regarding transportation and logistics. Furthermore, they are flexible and easy to handle. Therefore, they are in demand in the public health sector.

Another advantage of flexible plastic bags is that they are compatible with sterilisation by radiation, either by means of gamma rays or electron beam (E-beam). Currently there are solutions for hemoderivatives commercialized in plastic bags (e.g. Flexbumin^{®}, a 20% solution of human plasma albumin, traded by Baxalta Spain S.L.) available on the market.

Usually, said flexible plastic bags are sterilised prior to the filling thereof by means of irradiation with gamma rays or electron beam (E-beam). Sterilisation by radiation as ionising radiation is commonly used in hospitals for sanitary devices (e.g. catheters, surgical items, and critical care tools). Gamma irradiation is the most popular form of sterilisation by radiation and is typically used when the materials are sensitive to the high temperature of the autoclave.

In addition, it is increasingly common for rigid containers, also known as "nest" containers, to be used for sterilising surgical material, syringes, or vials. These containers are cost-effective, reusable, easy to transport and to clean, and are very effective in the sterilisation process. However, these known containers are not suitable for sterilising bags, due to the lack of rigidity and the fragility thereof.

For instance, the document of European Patent EP 3269409B1 discloses a container for sterilising syringes and the PCT application WO 2017/139385 A1 discloses a device for facilitating the correct position of syringes and a product inspection path. Although the two documents disclose containers for sterilising syringes, it is not possible to incorporate flexible bags in said containers for the sterilisation of said bags.

In addition, the document of Spanish Patent ES2248768T3 discloses a packaging intended to be used to transport sterile objects or objects to be sterilised, comprising a box intended to receive the sterile objects or objects to be sterilised and a cover sheet of selectively airtight material, which sheet is fixed above the box such that it will seal the latter in an airtight manner. This packaging is not suitable for flexible bags either.

It is also known, prior art describing "nest"-type systems for rigid containers. For example, US 2010/0307956 A1 discloses a device for storing, transporting, and protecting syringes suspended in a vertical position using a support plate with parallel rails and support lips forming grooves to retain syringes by their flanges. Likewise, US 2018/0134423 A10 discloses a "nest" for cartridges or cartridge closures, designed for filling and stoppering operations involving rigid container, it also discloses a method for closing medical cartridges using a closure nest and a cartridge nest and details about transport and packaging rigid containers for these structures. Neither of these packaging is suitable for flexible bags nor do they disclose retention means adapted to the port-plug region of such bags.

Therefore, there exists the need provide "nest" type containers for sterilising and transporting flexible plastic bags for pharmaceutical use, in particular flexible plastic bags that will contain human plasma byproducts. The use of this type of containers for sterilising and transporting flexible plastic bags is not known to the inventors of the present invention.

Surprisingly, the inventors of the present invention have developed a "nest" type container suitable for sterilising and transporting flexible plastic bags, which, in addition to the above advantages of these types of containers, allows said bags to be fastened by the plug region of said bags and is suitable for being filled both manually and automatically.

Therefore, in a first aspect, the present invention discloses a "nest" type container for sterilising flexible bags for pharmaceutical use, comprising:
a) an upper flat structure comprising at least one support element of said flexible bags which is designed to accommodate the port-plug structure of said flexible bag, said support element comprising at least one fastening element which is suitable for retaining said bags once they are inserted into said support element;
b) a lower receptacle which is designed to contain the upper flat structure (a) and to couple thereto in a detachable manner;
c) a cap for hermetically sealing said lower receptacle.

wherein said upper flat structure (a) is the cap (c) for hermetically sealing said lower receptacle and
wherein said container is made of high-density polyethylene (HDPE).

Preferably, the container of the present invention can accommodate between 1 and 250 flexible bags, more preferably between 1 and 180 flexible bags.

It is obvious to the skilled person that the shape of the container of the present invention can be any one suitable for sterilisation. Preferably, said container has the shape of a rectangular parallelepipedon.

Preferably, said support element of the flexible bags are bars with a T and/or L-shaped cross-section which are attached to the upper flat structure of the container of the present invention in an undetachable manner.

It is also preferable for said fastening element to be a semi-circle-shaped notch in said support elements. Said semi-circle must have a diameter greater than the port of the flexible bag but must be smaller than the diameter of the flange of the cap of said bag.

The invention will be explained below with reference to the figures, by way of explanatory, non-restrictive example, of various embodiments of the device of the present invention, in which:
Fig. 1 is a perspective view of an embodiment of the container for sterilising flexible bags of the present invention.
Fig. 2 is a front view of a first embodiment of the upper flat structure of the container for sterilising flexible bags of the present invention.
Fig. 3 is a perspective view of a second embodiment of the upper flat structure of the container for sterilising flexible bags of the present invention.
Fig. 4 is a perspective view of the first embodiment of the upper flat structure of the container for sterilising flexible bags of the present invention.
Fig. 5 is a perspective view of the lower part of the first embodiment of the upper flat structure of the container for sterilising flexible bags of the present invention, shown in Fig. 4.
Fig. 6 is a perspective view of the first embodiment of the upper flat structure of the container for sterilising flexible bags of the present invention, a bag having been placed.
Fig. 7 is a perspective view of the lower part of the first embodiment of the upper flat structure of the container for sterilising flexible bags of the present invention, the bags being alternately placed.
Fig. 8 is a perspective view of the lower part of the first embodiment of the upper flat structure of the container for sterilising flexible bags of the present invention, all the bags to be sterilised having been placed.
Fig. 9 is a perspective view of an embodiment of the container of the present invention when hermetically sealed.

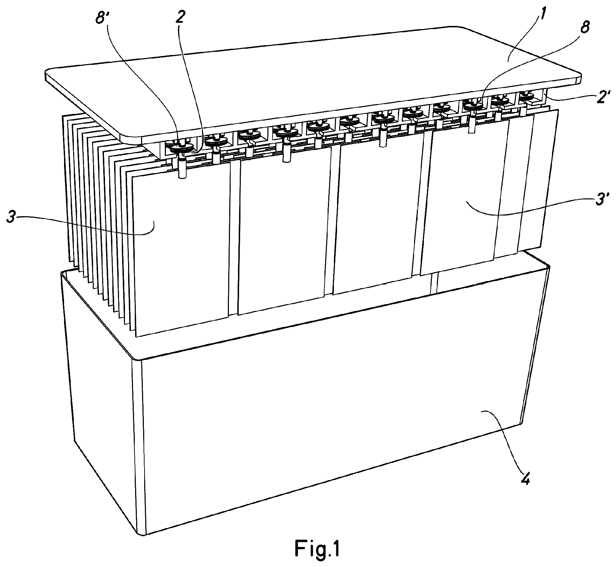

As seen in Fig. 1, the container for sterilising flexible bags of the present invention is formed by an upper flat structure -1-, in which the lower part has the support elements -2-, -2'- of said flexible bags -3-, -3'-, which elements are designed to accommodate the port-plug structure -8-, -8'- of said flexible bags -3-, -3'-, and a lower receptacle -4- in the shape of a rectangular parallelepipedon which is designed to accommodate the upper flat structure -1-, which in this case also acts as a cap for hermetically sealing said lower receptacle -4-.

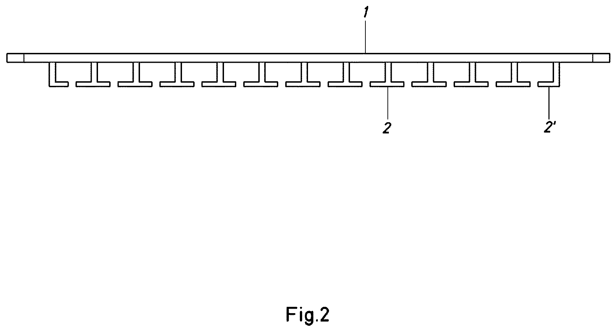

Fig. 2 shows a first embodiment of the upper flat structure -1-, in which the lower part thereof has support elements -2-, -2'- of said flexible bags.

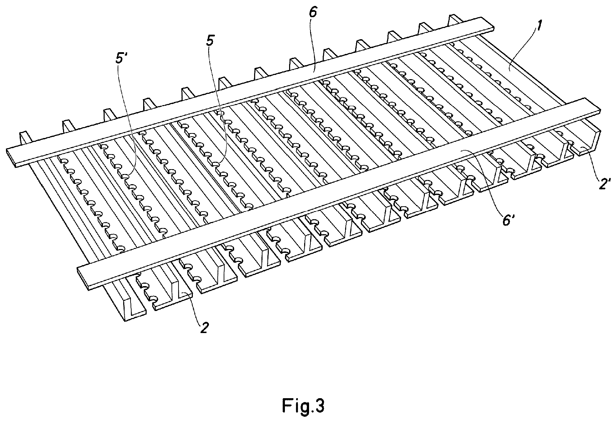

Fig. 3 is a perspective view of a second embodiment of the upper flat structure -1- of the container for sterilising flexible bags of the present invention. It also shows that the support elements -2-, -2'- are attached to each other by two bars -6-, -6'- parallel to each other and perpendicular to said support elements -2-, -2'-. It also shows the fastening elements -5-, -5'- of the flexible bags in the shape of semicircles arranged in the support elements -2-, -2'-.

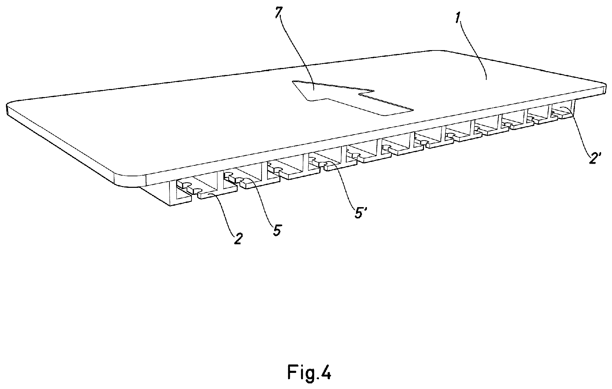

In addition, Fig. 4 is a perspective view of the first embodiment of the upper flat structure of the container for sterilising flexible bags of the present invention. In said figure, the upper flat structure -1- is also the cap for the hermetically sealing the lower receptacle of the container of the present invention. The support elements -2-, -2'-, which comprise the fastening elements -5-, -5'-, have the flexible bags in the lower part of said upper flat structure -1-. The arrow -7- indicates the direction in which the flexible bags will be placed in the upper flat structure -1- of the container of the present invention.

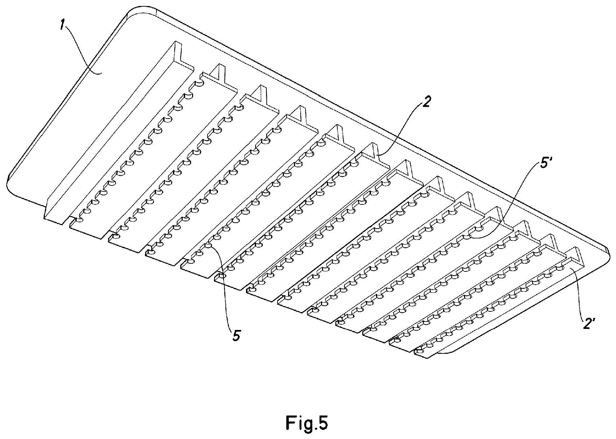

Fig. 5 is a perspective view of the lower part of the first embodiment of the upper flat structure of Fig. 4. It is noted how some of the support elements -2 can have the T-shaped cross-section and others -2'- have the L-shaped cross-section.

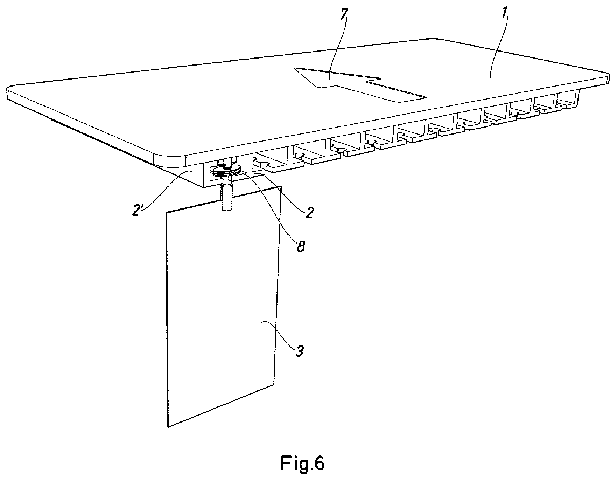

Fig. 6 is a perspective view of the first embodiment of the upper flat structure -1- of the container for sterilising flexible bags of the present invention, a flexible bag -3- having been placed. The port-plug structure -8- of said flexible bag -3- is inserted into the slot formed by the support elements -2-, -2'- and is moved in the direction of the arrow -7- as far as the furthermost fastening element (not shown in the figure) of the support elements -2-, -2'- through which said flexible bag -3- was inserted.

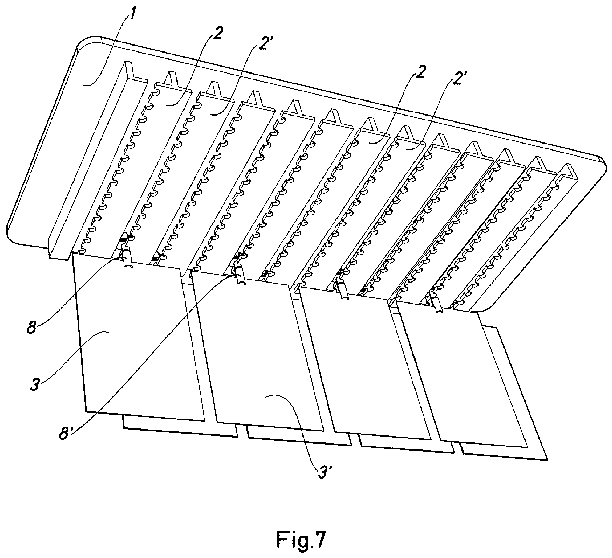

Fig. 7 is a perspective view of the first embodiment of the lower part of the upper flat structure -1- of the container for sterilising flexible bags of the present invention, said flexible bags -3-, -3'- being alternately placed and the container being partially full. The port-plug structures -8-, -8'- of said flexible bags -3-, -3'-, after being inserted into the slots formed by the support elements -2-, -2'-, are accommodated in the fastening elements of the upper flat structure -1-.

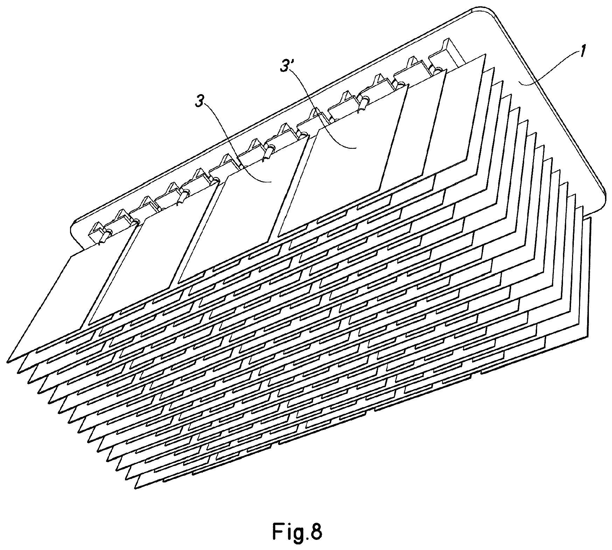

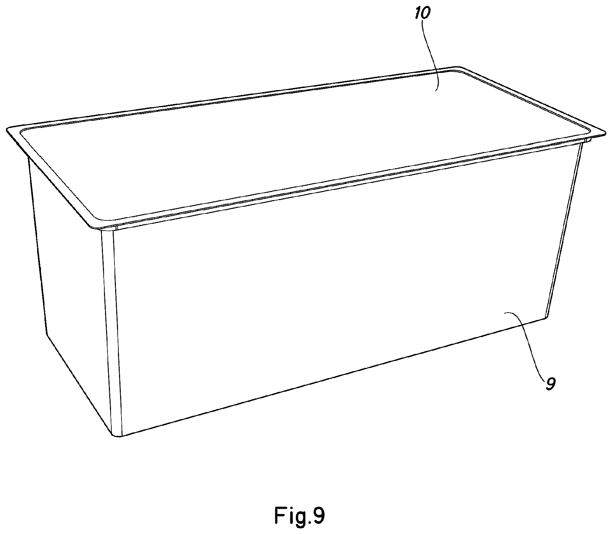

Fig. 8 is a perspective view of the first embodiment of the lower part of the upper flat structure -1- of the container for sterilising flexible bags of the present invention, all the flexible bags -3-, -3'- to be sterilised being placed in the upper flat structure -1-. Said upper flat structure is inserted into the lower receptacle -9- in order to obtain a container -10- for sterilising said bags (as shown in Fig. 9), which is hermetically sealed for sterilising and transporting the flexible bags arranged therein.

## Claims

1. Container (10) for sterilising flexible bags (3, 3') for pharmaceutical use, **characterised in that** it comprises:
a) an upper flat structure (1) comprising at least one support element (2, 2') of said flexible bags (3, 3'), which support element (2, 2') is designed to accommodate the port-plug structure (8, 8') of said flexible bag (3, 3'), said support element (2, 2') comprising at least one fastening element (5, 5') suitable for retaining said flexible bags (3, 3') once they are inserted into said support element (2, 2');
b) a lower receptacle (4) designed to contain said upper flat structure (1) and to couple thereto in a detachable manner; and c) a cap (1) for hermetically sealing said lower receptacle (4)
wherein said upper flat structure (1) is the cap (1) for hermetically sealing said lower receptacle (4) and wherein said container (10) is made of high-density polyethylene (HDPE).

2. Container (10) for sterilising flexible bags (3, 3') for pharmaceutical use according to claim 1, **characterised in that** it may contain from 1 to 250 flexible bags (3, 3').

3. Container (10) for sterilising flexible bags (3, 3') for pharmaceutical use according to claim 2, **characterised in that** it may contain from 1 to 180 flexible bags (3, 3').

4. Container (10) for sterilising flexible bags (3, 3') for pharmaceutical use according to any of the claims above, **characterised in that** said container (10) has the shape of a rectangular parallelepipedon.

5. Container (10) for sterilising flexible bags (3, 3') for pharmaceutical use according to any of the claims above, **characterised in that** said support element (2, 2') of the flexible bags (3, 3') are bars with a T and/or L-shaped crosssection which are attached in a non-detachable manner to the upper flat structure (1) of the container (10).

## Patentansprüche

1. Behälter (10) zum Sterilisieren flexibler Beutel (3, 3') für eine pharmazeutische Verwendung, **dadurch gekennzeichnet, dass** er umfasst
a) eine obere flache Struktur (1), umfassend mindestens ein Stützelement (2, 2') der genannten flexiblen Beutel (3, 3'), welches Stützelement (2, 2') ausgelegt ist, die Anschluss-Stopfen-Struktur (8, 8') des genannten flexiblen Beutels (3, 3') aufzunehmen, wobei das genannte Stützelement (2, 2') mindestens ein Befestigungselement (5, 5') umfasst, das geeignet ist, die genannten flexiblen Beutel (3, 3') zu halten, sobald sie in das genannte Stützelement (2, 2') eingesetzt sind;
b) ein unteres Behältnis (4), das ausgelegt ist, die genannte obere flache Struktur (1) zu enthalten und mit dieser in lösbarer Weise gekoppelt zu werden; und
c) einen Deckel (1) zum hermetischen Verschließen des genannten unteren Behältnisses (4), wobei die genannte obere flache Struktur (1) der Deckel (1) zum hermetischen Verschließen des genannten unteren Behältnisses (4) ist und wobei der genannte Behälter (10) aus hochdichtem Polyethylen (HDPE) hergestellt ist.

2. Behälter (10) zum Sterilisieren flexibler Beutel (3, 3') für eine pharmazeutische Verwendung nach Anspruch 1, **dadurch gekennzeichnet, dass** er 1 bis 250 flexible Beutel (3, 3') enthalten kann.

3. Behälter (10) zum Sterilisieren flexibler Beutel (3, 3') für eine pharmazeutische Verwendung nach Anspruch 2, **dadurch gekennzeichnet, dass** er 1 bis 180 flexible Beutel (3, 3') enthalten kann.

4. Behälter (10) zum Sterilisieren flexibler Beutel (3, 3') für eine pharmazeutische Verwendung nach einem der vorhergehenden Ansprüche, **dadurch gekennzeichnet, dass** der genannte Behälter (10) die Form eines rechtwinkligen Parallelepipeds aufweist.

5. Behälter (10) zum Sterilisieren flexibler Beutel (3, 3') für eine pharmazeutische Verwendung nach einem der vorhergehenden Ansprüche, **dadurch gekennzeichnet, dass** das genannte Stützelement (2, 2') der flexiblen Beutel (3, 3') Stäbe mit einem T- und/oder L-förmigen Querschnitt sind, die in nicht lösbarer Weise an der oberen flachen Struktur (1) des Behälters (10) angebracht sind.

## Revendications

1. Récipient (10) destiné à la stérilisation de sachets souples (3, 3') à usage pharmaceutique, **caractérisé en ce qu'**il comprend :
a) une structure supérieure plane (1) comprenant au moins un élément de support (2, 2') desdits sachets souples (3, 3'), lequel élément de support (2, 2') est conçu pour recevoir la structure d'obturation (8, 8') dudit sachet souple (3, 3'), ledit élément de support (2, 2') comprenant au moins un élément de fixation (5, 5') adapté pour retenir lesdits sachets souples (3, 3') une fois qu'ils sont insérés dans ledit élément de support (2, 2') ;
b) un réceptacle inférieur (4) conçu pour contenir ladite structure supérieure plane (1) et pour s'y accoupler de manière amovible ; et c) un capuchon (1) destiné à fermer hermétiquement ledit réceptacle inférieur (4)
dans lequel ladite structure supérieure plane (1) est le capuchon (1) destiné à fermer hermétiquement ledit réceptacle inférieur (4) et dans lequel ledit récipient (10) est réalisé en polyéthylène haute densité (PEHD).

2. Récipient (10) destiné à la stérilisation de sachets souples (3, 3') à usage pharmaceutique selon la revendication 1, **caractérisé en ce qu'**il peut contenir de 1 à 250 sachets souples (3, 3').

3. Récipient (10) destiné à la stérilisation de sachets souples (3, 3') à usage pharmaceutique selon la revendication 2, **caractérisé en ce qu'**il peut contenir de 1 à 180 sachets souples (3, 3').

4. Récipient (10) destiné à la stérilisation de sachets souples (3, 3') à usage pharmaceutique selon l'une quelconque des revendications précédentes, **caractérisé en ce que** ledit conteneur (10) a la forme d'un parallélépipède rectangle.

5. Récipient (10) destiné à la stérilisation de sachets souples (3, 3') à usage pharmaceutique selon l'une quelconque des revendications ci-dessus, **caractérisé en ce que** lesdits éléments de support (2, 2') des sachets souples (3, 3') sont des barres de section transversale en forme de T et/ou de L qui sont fixées de manière indétachable à la structure supérieure plane (1) du récipient (10).
